# EUROPÄISCHE PATENTANMELDUNG

(11) **EP 4 527 565 A1**
(43) Veröffentlichungstag der Anmeldung: **26.03.2025**
(21) Anmeldenummer: 24198556.3
(22) Anmeldetag: 05.09.2024
(51) Int. Cl.: B25J 13/02, A61B 34/00, G06F 3/01

(54) **HAPTISCHE VORRICHTUNG**

(30) Priorität: 21.09.2023 DE 102023125682
(71) Anmelder: Karl Storz SE & Co. KG, 78532 Tuttlingen (DE)
(72) Erfinder: Hoffmann, Martin, 78532 Tuttlingen (DE); Klingels, Torben, 78532 Tuttlingen (DE)

(57) **Zusammenfassung**

Die vorliegende Erfindung bezieht sich auf eine haptische Vorrichtung (1, 1') mit einem Handstück (10), einer Basisstruktur (5) und einer Handgelenkstruktur, die das Handstück (10) mit der Basisstruktur (5) verbindet und drei rotatorische Freiheitsgrade des Handstücks (10) in Bezug auf die Basisstruktur (5) bereitstellt. Die Handgelenkstruktur weist eine Kardanikvorrichtung (2), eine erste Betätigungsstange (6) und einen Grundarm (4) auf, wobei die Kardanikvorrichtung (2) in dem Handstück (10) aufgenommen ist und zwei orthogonale, sich schneidende Schwenkachsen (A, B) aufweist. Dabei stellt eine erste Schwenkachse (A) einen ersten rotatorischen Freiheitsgrad (r) des Handstücks (10) bereit, und eine zweite Schwenkachse (B) einen zweiten rotatorischen Freiheitsgrad (g) des Handstücks (10). Die erste Betätigungsstange (6) definiert eine Längsachse (C) und ist an einem Kopplungsende (6.2) mit der Kardanikvorrichtung (2) verbunden, wobei die erste Betätigungsstange (6) um ihre Längsachse (C) drehbar ist und einen dritten rotatorischen Freiheitsgrad (n) des Handstücks (10) bereitstellt. Der Grundarm (4) ist mit der Basisstruktur (5) verbunden und weist einen Halterungsabschnitt (4.1) auf, der die erste Betätigungsstange (6) in einem vorbestimmten Abstand zu dem Kopplungsende (6.2) drehbar gelagert hält.

## Beschreibung

Die Erfindung betrifft eine haptische Vorrichtung, die eine Schnittstelle zwischen Mensch und technischem System bereitstellt.

Aus dem Stand der Technik sind haptische Vorrichtungen mit einem Stellteil als Verbindungs- bzw. Eingabeschnittstelle zwischen Nutzer und einem technischen System bekannt, um z. B. in Roboteranwendungen oder Teleoperationen eine Handbewegung in das System einzugeben. Üblicherweise stellt eine derartige haptische Vorrichtung zumindest drei Bewegungsmöglichkeiten bzw. Freiheitsgrade bereit, die Translations- und/oder Rotationsbewegungen im Raum umfassen.

Solche haptischen Vorrichtungen können als passive Systeme zur reinen Orientierungserfassung ausgebildet sein, wobei vorliegend unter dem Begriff "passiv" verstanden wird, dass Kräfte und/oder Drehmomente, mit denen ein Nutzer das Stellteil bewegt, eine entsprechende Bewegung des technischen Systems hervorrufen können. Alternativ können solche haptischen Vorrichtungen aber auch für Force-Feedback bzw. Kraftrückkopplung als aktive Systeme ausgebildet sein, die eine vom Nutzer über das Stellteil erfahrbare Kraft als Rückmeldung einsetzen. Die dem Benutzer an dem Stellteil angezeigten Kräfte und/oder Drehmomente werden mittels kontrollierter Energiezufuhr von einem oder mehreren Aktoren erzeugt.

Aus WO 2008/003417 A1 ist eine haptische Eingabevorrichtung bekannt, die einen mit einem Handgriff verbundenen Endeffektor aufweist, der über eine parallelkinematische Struktur mit einem Basisteil verbunden ist, wobei die parallelkinematische Struktur translatorische Freiheitsgrade in Bezug auf den Endeffektor bereitstellt. Der Handgriff ist entweder einfach drehbar mit dem Endeffektor verbunden, sodass der Handgriff einen rotatorischen Freiheitsgrad in Bezug auf den Endeffektor aufweist, oder der Handgriff kann mit dem Endeffektor über eine Handgelenkstruktur verbunden sein, die drei rotatorische Freiheitsgrade in Bezug auf den Endeffektor bereitstellt. Dazu weist die Handgelenkstruktur ein Gestänge mit zwei gewinkelten Profilen auf, die schwenkbar miteinander und einerseits mit dem Endeffektor und andererseits mit dem Handgriff verbunden sind, sodass die Schwenkachsen benachbarter Schwenkgelenke orthogonal zueinander sind, und sich die drei Schwenkachsen in einem gemeinsamen Rotationszentrum schneiden, das innerhalb des Handgriffs liegt. Somit umgibt die Handgelenkstruktur bei Bedienung am Handgriff die Hand des Benutzers, was deren Bewegungsbereich einschränken und zu Kollisionen zwischen der Handgelenkstruktur und der Hand des Benutzers oder zwischen den Eingabegeräten und/oder der Umgebung führen kann.

Ein Problem bei Handgelenkstrukturen mit drei rotatorischen Freiheitsgraden für den Handgriff besteht im sogenannten "Gimbal Lock", was einen kritischen Zustand eine kardanische Aufhängung bezeichnet, wenn zwei der drei Schwenkachsen parallel zueinander zu liegen kommen, sodass ein Freiheitsgrad wegfällt und bestimmte Ausrichtungen nicht erreicht werden können. Im Falle einer Handgelenkstruktur bedeutet dies, dass Drehungen des Handgriffs um eine Achse, die orthogonal sowohl auf der ersten Schwenkachse als auch auf der zweiten Schwenkachse steht, blockiert sind, wenn die erste Schwenkachse und die dritte Schwenkachse parallel zueinander stehen.

Ausgehend von diesem Stand der Technik ist es Aufgabe der vorliegenden Erfindung, eine verbesserte haptische Eingabevorrichtung bereitzustellen, die einen großen Bewegungsbereich ohne Gimbal-Lock zulässt.

Diese Aufgabe wird durch eine haptische Vorrichtung mit den Merkmalen des Anspruchs 1 gelöst.

Weiterbildungen bzw. bevorzugte Ausführungsformen sind in den Unteransprüchen ausgeführt.

Die erfindungsgemäße haptische Vorrichtung weist nach einer ersten Ausführungsform ein Handstück, eine Basisstruktur und eine Handgelenkstruktur auf, die das Handstück mit der Basisstruktur verbindet und drei rotatorische Freiheitsgrade des Handstücks in Bezug auf die Basisstruktur bereitstellt. Vorteilhaft weist die Handgelenkstruktur eine Kardanikvorrichtung auf, die in dem Handstück aufgenommen ist und die zwei orthogonale, sich schneidende Schwenkachsen aufweist, wobei eine erste Schwenkachse einen ersten rotatorischen Freiheitsgrad des Handstücks bereitstellt, und eine zweite Schwenkachse einen zweiten rotatorischen Freiheitsgrad des Handstücks bereitstellt. Die Handgelenkstruktur weist eine erste Betätigungsstange auf, die eine Längsachse definiert und an einem Kopplungsende mit der Kardanikvorrichtung verbunden ist, wobei die erste Betätigungsstange um ihre Längsachse drehbar ist und einen dritten rotatorischen Freiheitsgrad des Handstücks bereitstellt. Ferner weist die Handgelenkstruktur einen Grundarm auf, der mit der Basisstruktur verbunden ist und einen Halterungsabschnitt aufweist, der die erste Betätigungsstange in einem vorbestimmten, d. h. bei der Montage festgelegten, Abstand zu dem Kopplungsende drehbar gelagert hält, sodass durch die Form und Länge des unbeweglichen Grundarms eine Lage und Ausrichtung des Handstücks in Bezug zu der Basisstruktur festgelegt ist.

Die erfindungsgemäße haptische Vorrichtung, die eine vergleichsweise kompakte und leichte Konstruktion aufweisen kann, lässt einen großen Bewegungsbereich ohne Gimbal-Lock zu, wobei eine Hand des Benutzers, die das Handstück greift, nicht durch ein Gestänge umschlossen wird, sodass ein versehentlicher Kontakt der Hand des Benutzers mit einem Gestänge der Vorrichtung beim Ergreifen und Loslassen des Handstücks praktisch kaum stattfindet. Zudem ist die Kollisionsgefahr mit umgebenden Strukturen oder - bei Verwendung von zwei haptischen Vorrichtungen für die rechte und die linke Hand - zwischen den beiden Vorrichtungen deutlich reduziert. Das Handstück der erfindungsgemäßen haptischen Vorrichtung kann dabei als Stellteil zur passiven Orientierungserfassung oder zur aktiven Kraftrückkopplung eingesetzt werden.

Vorliegend werden teilweise auch die Bezeichnungen Rollen, Gieren und Nicken zur Unterscheidung der rotatorischen Freiheitsgrade verwendet, wobei der erste rotatorische Freiheitsgrad, also die Drehmöglichkeit um die erste Schwenkachse, als Rollen, und der zweite rotatorischen Freiheitsgrad, also die Drehmöglichkeit um die zweite Schwenkachse, als Gieren, und der dritte rotatorische Freiheitsgrad, die Drehmöglichkeit um die Längsachse, als Nicken bezeichnet werden. Befindet sich das Handstück in neutraler Lage, erstreckt sich die Längsachse orthogonal zu der Ebene, in der die erste und zweite Schwenkachse liegen.

Nach einer weiteren Ausführungsform der erfindungsgemäßen haptischen Vorrichtung wird die erste Schwenkachse der Kardanikvorrichtung durch eine Kreuzstrebe bereitgestellt, die drehbar in dem Handstück gelagert ist. Die zweite Schwenkachse wird dann durch ein Zylinderstück bereitgestellt, durch das sich die Kreuzstrebe orthogonal erstreckt und - zur drehbaren Lagerung in dem Handstück - mit beiden Enden diametral aus dem Zylinderstück hinausragt. Somit entsprechen die Längsachsen der Kreuzstrebe und des Zylinderstücks der ersten und zweiten Schwenkachse. Dabei ist das Kopplungsende der ersten Betätigungsstange als Kugelabschnitt ausgebildet, der zum einen eine Zylinderbohrung aufweist, in der das Zylinderstück drehbar angeordnet ist. Zum anderen weist der Kugelabschnitt zwei diametrale kreisbogenartige Nutsegmente auf, die sich entlang einer zu der Zylinderbohrung orthogonalen Ebene erstrecken, die durch einen Mittelpunkt des Kugelabschnitts verläuft, wobei die über das Zylinderstück hinausragenden Enden der Kreuzstrebe in den Nutsegmenten aufgenommen sind.

Ferner kann eine erfindungsgemäße haptische Vorrichtung gemäß einer weiteren Ausführungsform vorsehen, dass die Kardanikvorrichtung eine Kugelpfannenhülse aufweist, die zur drehbaren Lagerung der Kreuzstrebe in dem Handstück ausgebildet und dazu drehfest in dem Handstück angeordnet ist. Dabei ist der das Kopplungsende bildende Kugelabschnitt der ersten Betätigungsstange in der Kugelpfannenhülse gelenkig aufgenommen.

Grundsätzlich ist es denkbar, dass die drehbare Lagerung der erste Betätigungsstange mittels eines Lagers in dem Halterungsabschnitt erfolgt, zur verbesserten Stabilität und Führung ist allerdings nach einer bevorzugten Ausführungsform der erfindungsgemäßen haptischen Vorrichtung vorgesehen, dass die erste Betätigungsstange drehbar in einem Stangengehäuse gelagert ist, das in dem vorbestimmten Abstand zu dem Kopplungsende mit dem Halterungsabschnitt des Grundarms verbunden ist.

Alternative oder zusätzliche Ausführungsformen der erfindungsgemäßen haptischen Vorrichtung sehen vor, dass der Grundarm einen gebogenen und/oder gewinkelten Verlauf aufweist, um eine gewünschte Ausrichtung des Handstücks in Bezug zur Basisstruktur zu erreichen. Dazu kann der Grundarm zusätzlich zu dem Halterungsabschnitt zumindest einen weiteren Armabschnitt haben, der starr mit dem Halterungsabschnitt verbunden ist, wobei der Halterungsabschnitt und der zumindest eine weitere Armabschnitt zueinander gewinkelt verlaufen und/oder jeweils eine gerade oder gebogene Form aufweisen. Durch alternative Gestaltung des Grundarms bei der Konstruktion der haptischen Vorrichtung ist es möglich, zusammen mit dem in der Montage festlegbaren Abstand zwischen dem Kopplungsende der Betätigungsstange und dem Halterungsabschnitt, die Lage und Ausrichtung des Handstücks in Bezug zu der Basisstruktur je nach vorgesehener Anwendung zu variieren.

Eine weitere Ausführungsform der erfindungsgemäßen haptischen Vorrichtung bezieht sich darauf, dass die haptische Vorrichtung als haptische Vorrichtung mit Kraftrückkopplung, d. h. zur aktiven Kraftrückmeldung über das Handstück an den Benutzer ausgebildet ist. Dabei ist die erste Betätigungsstange an einem von dem Kopplungsende abgewandten Ende mit einem ersten Aktor verbunden, um eine Drehbewegung der ersten Betätigungsstange mit dem Handstück um die Längsachse entsprechend dem dritten rotatorischen Freiheitsgrad (Nicken) zu betätigen, wobei der erste Aktor an dem Grundarm angeordnet ist. Unter Anordnung des Aktors an dem Grundarm soll verstanden werden, dass der Aktor statisch in Bezug auf den Grundarm vorliegt, wobei der Aktor ggf. mittels einer Halterung mit dem Grundarm verbunden sein kann, aber nicht muss. Es ist auch möglich, dass der Aktor ggf. mit einer Halterung an der Basisstruktur oder einer weiteren Komponente der haptischen Vorrichtung befestigt ist, ohne direkt mit dem Grundarm verbunden zu sein.

Nach weiteren Ausführungsformen kann die erfindungsgemäße haptischen Vorrichtung mit Kraftrückkopplung einen zweiten Aktor zur Betätigung einer Drehbewegung der Kardanikvorrichtung und des Handstücks um die erste Schwenkachse entsprechend dem ersten rotatorischen Freiheitsgrad (Rollen) oder einen dritten Aktor zur Betätigung einer Drehbewegung der Kardanikvorrichtung um die zweite Schwenkachse entsprechend dem zweiten rotatorischen Freiheitsgrad (Gieren) aufweisen. Dabei sind der zweite Aktor über eine erste Kraftübertragungsvorrichtung und der dritte Aktor über eine zweite Kraftübertragungsvorrichtung mit der Kardanikvorrichtung operativ gekoppelt und an dem Grundarm angeordnet, d. h. wie der erste Aktor statisch in Bezug auf den Grundarm.

Eine bevorzugte Ausführungsform der erfindungsgemäßen haptischen Vorrichtung mit Kraftrückkopplung sieht vor, dass diese sowohl den zweiten als auch den dritten Aktor aufweist, um mittels der ersten und zweiten Kraftübertragungsvorrichtung eine Drehbewegung der Kardanikvorrichtung und des Handstücks um die erste und die zweite Schwenkachse betätigen zu können, sodass die Kraftrückkopplung alle drei Freiheitsgrade, Nicken, Rollen und Gieren, umfasst. Durch die statische Anordnung aller Aktoren bleiben vorteilhaft auch bei der Ausführung der haptischen Vorrichtung mit Kraftrückkopplung die gute Zugänglichkeit des Handstücks und die kollisionsfreie Bedienbarkeit erhalten.

Ferner kann eine erfindungsgemäße haptische Vorrichtung mit Kraftrückkopplung nach einer weiteren Ausführungsform einen Befestigungskragen zur operativen Kopplung der ersten Kraftübertragungsvorrichtung oder der zweiten Kraftübertragungsvorrichtung oder beider Kraftübertragungsvorrichtungen mit der Kardanikvorrichtung aufweisen. Dazu wird der Befestigungskragen, der die Kardanikvorrichtung, bzw. die Kugelpfannenhülse der Kardanikvorrichtung, ringförmig umgibt, in dem Handstück angeordnet und gelenkig mit der ersten und/oder zweiten Kraftübertragungsvorrichtung verbunden. Bevorzugt ist der Befestigungskragen mit beiden Kraftübertragungsvorrichtungen gelenkig verbunden. Die Anordnung eines Lagers sorgt für eine Entkopplung des Befestigungskragens von einer Drehbewegung der Kardanikvorrichtung und des Handstücks um die Längsachse entsprechend dem dritten rotatorischen Freiheitsgrad (Nicken).

Nach einer Weiterbildung der erfindungsgemäßen haptischen Vorrichtung mit Kraftrückkopplung kann die gelenkige Verbindung des Befestigungskragens mit den beiden Kraftübertragungsvorrichtungen eine erste Gelenkverbindung der ersten Kraftübertragungsvorrichtung und eine zweite Gelenkverbindung der zweiten Kraftübertragungsvorrichtung aufweisen, wobei eine der beiden Gelenkverbindungen, die die erste Gelenkverbindung und die zweite Gelenkverbindung umfassen, einen Freiheitsgrad und die andere der beiden Gelenkverbindungen zwei Freiheitsgrade bereitstellt, sodass vollständige Bewegungsfreiheit des Befestigungskragens gewährleistet ist, ohne dass das System unbestimmt wird.

Nach weiteren Ausführungsformen der erfindungsgemäßen haptischen Vorrichtung kann vorgesehen sein, dass die erste und/oder die zweite Kraftübertragungsvorrichtung jeweils eine mechanische Kraftübertragungsvorrichtung ist, die aus einer Gruppe ausgewählt ist, die ein Schubstangengestänge und ein Kabelzugsystem umfasst und weitere gängige mechanische Kraftübertragungsvorrichtungen aufweisen kann.

So können nach einer speziellen Ausführungsform der erfindungsgemäßen haptischen Vorrichtung die erste und die zweite Kraftübertragungsvorrichtung durch ein erstes und ein zweites Schubstangengestänge bereitgestellt werden: Dabei weist das erste Schubstangengestänge eine zur Längsachse koaxiale, hohlzylindrische zweite Betätigungsstange auf, die in Längsrichtung beweglich um die erste Betätigungsstange angeordnet ist. Das zweite Schubstangengestänge weist entsprechend eine zur Längsachse koaxiale, hohlzylindrische dritte Betätigungsstange auf, die in Längsrichtung beweglich um die zweite Betätigungsstange angeordnet ist, wobei die zweite Betätigungsstange an beiden Enden aus der dritten Betätigungsstange hinausragt. D. h., dass die Längen der Betätigungsstangen von innen nach au-ßen abnehmen - die erste Betätigungsstange hat die größte Länge, die zweite Betätigungsstange eine mittlere Länge und die dritte Betätigungsstange die kürzeste Länge.

Die zweite (mittlere) Betätigungsstange ist an ihrem handstückfernen Ende über ein erstes Exzentergestänge mit dem zweiten Aktor verbunden, und die dritte (äußerste) Betätigungsstange ist an ihrem handstückfernen Ende über ein zweites Exzentergestänge mit dem dritten Aktor verbunden. Das erste und das zweite Exzentergestänge sind dabei in üblicher Weise mit mehreren gelenkig verbundenen Gestängeabschnitten zur Übersetzung einer rotatorischen Bewegung des zweiten bzw. dritten Aktors in eine lineare Bewegung der zweiten bzw. dritten Betätigungsstange ausgebildet.

Am anderen Ende, d. h. an ihrem handstücknahen Ende, weist die zweite Betätigungsstange einen sich in radialer Richtung erstreckenden ersten Kopplungsabschnitt auf, der über eine erste Gelenkverbindung mit einem ersten Übertragungsabschnitt verbunden ist, der beabstandet zu der zweiten Betätigungsstange angeordnet ist und über ein erstes Kugelgelenk mit der Kardanikvorrichtung, bzw. dem Befestigungskragen der Kardanikvorrichtung, verbunden ist. Auch die dritte Betätigungsstange weist an ihrem handstücknahen Ende einen zweiten Kopplungsabschnitt auf, der sich ebenfalls in radialer Richtung, allerdings um 90° versetzt zu dem ersten Kopplungsabschnitt erstreckt und über eine zweite Gelenkverbindung mit einem zweiten Übertragungsabschnitt verbunden ist, der beabstandet zu der dritten Betätigungsstange angeordnet ist und über ein zweites Kugelgelenk mit der Kardanikvorrichtung, bzw. dem Befestigungskragen der Kardanikvorrichtung, verbunden ist.

Hierbei kann die erste Gelenkverbindung die Gelenkverbindung mit einem Freiheitsgrad zur Kopplung des Befestigungskragens mit der ersten Kraftübertragungsvorrichtung und die zweite Gelenkverbindung die Gelenkverbindung mit zwei Freiheitsgraden zur Kopplung des Befestigungskragens mit der zweiten Kraftübertragungsvorrichtung sein, die die vollständige Bewegungsfreiheit des Befestigungskragens gewährleisten, ohne dass das System unbestimmt wird.

Nach einer weiteren Ausführungsform der erfindungsgemäßen haptischen Vorrichtung können die zweite und die dritte Betätigungsstange zusammen mit der ersten Betätigungsstange in dem Stangengehäuse angeordnet sein. Das Stangengehäuse weist dabei an einem handstücknahen Abschnitt in Längsrichtung zumindest einen Längsschlitz auf, durch den sich der erste und der zweite Kopplungsabschnitt erstrecken. Dabei kann es sich um einen breiten Schlitz handeln, der sich über 90° erstreckt, sodass sich beide Kopplungsabschnitte, die orthogonal zueinander stehen, durch einen gemeinsamen Schlitz erstrecken. Oder im Stangengehäuse sind zwei um 90° versetze Schlitze ausgebildet, in denen jeweils einer der Kopplungsabschnitte geführt wird. In Abhängigkeit der Längenverhältnisse der drei Betätigungsstangen und des jeweiligen axialen Betätigungswegs der zweiten und dritten Betätigungsstange können spezielle Ausführungsformen zudem vorsehen, dass die dritte Betätigungsstange an einem handstücknahen Abschnitt in Längsrichtung einen Längsschlitz aufweist, in dem der erste Kopplungsabschnitt aufnehmbar bzw. führbar ist.

Ferner bezieht sich eine Ausführungsform der erfindungsgemäßen haptische Vorrichtung unabhängig von ihrem Einsatz zur passiven Orientierungserfassung oder zur aktiven Kraftrückkopplung darauf, dass der Grundarm unbeweglich oder drehbar um eine zusätzliche Rotationsachse mit der Basisstruktur verbunden ist, wobei die Basisstruktur aus einer Gruppe ausgewählt ist, die eine Basisstruktur zur ortsfesten Anordnung im Raum und eine als Endeffektor ausgebildete Basisstruktur zur Anordnung an einem Ende einer Positionierungsvorrichtung umfasst. Eine Positioniervorrichtung kann z. B. einer Kinematik zur Bereitstellung translatorischer Freiheitsgrade in Bezug auf die als Endeffektor ausgebildete Basisstruktur sein.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- Fig. 1: eine perspektivische Ansicht der haptischen Vorrichtung nach einer erfindungsgemäßen Ausführungsform als passives System ohne Aktoren,
- Fig. 2: eine perspektivische Ansicht der haptischen Vorrichtung nach einer erfindungsgemäßen Ausführungsform mit Kraftrückkopplung,
- Fig. 3: eine Längsschnittansicht der haptischen Vorrichtung aus Fig. 2 mit Handstück in neutraler Lage,
- Fig. 4: eine perspektivische Ansicht der haptischen Vorrichtung aus Fig. 2 mit "nach innen" gerolltem Handstück,
- Fig. 5: eine perspektivische Ansicht der haptischen Vorrichtung aus Fig. 2 mit "nach außen" gerolltem Handstück,
- Fig. 6: eine Längsschnittansicht der haptischen Vorrichtung aus Fig. 3 mit "nach rechts" gegiertem Handstück.

Die vorliegende Erfindung bezieht sich auf eine haptische Vorrichtung mit einem Handstück als Stellteil, um zur Steuerung eines technischen Systems eine Orientierung des Stellteils im Raum einzugeben und/oder eine taktile Rückmeldung auf eine Interaktion mit dem technischen System zu erhalten. Die erfindungsgemäße haptische Vorrichtung ermöglicht eine Drehung des Handstücks um drei Achsen, ohne dass eine Gimbal-Sperre auftreten kann.

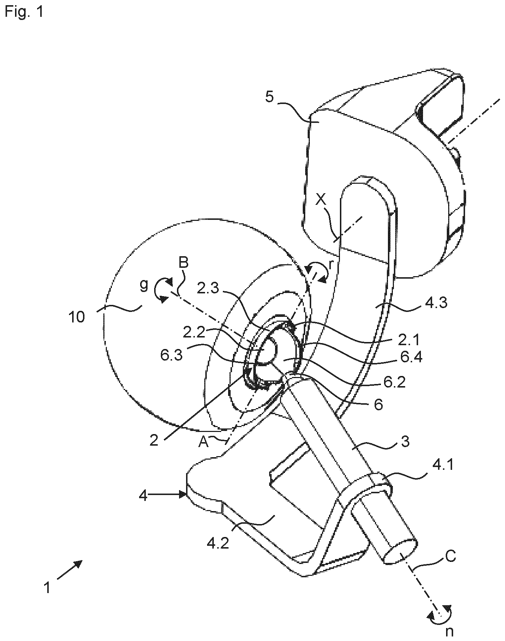

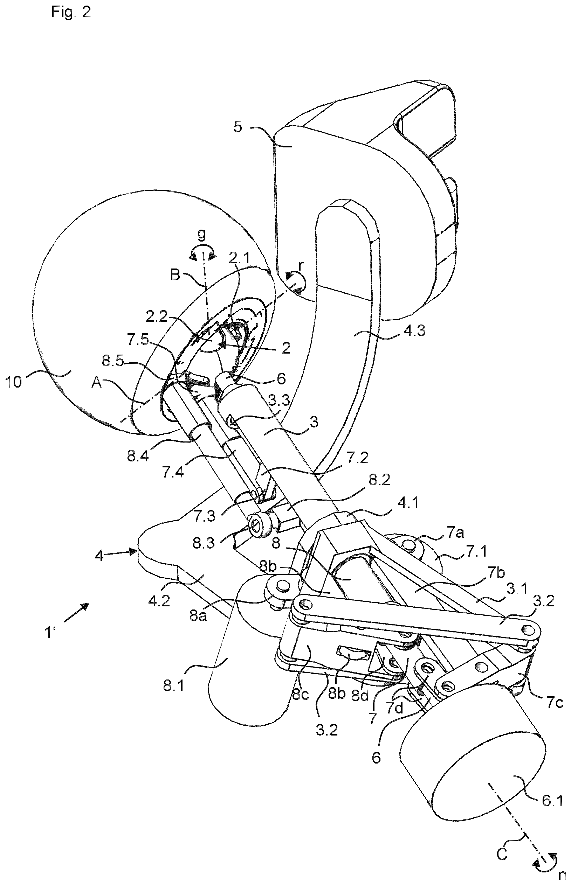

Fig. 1 zeigt die haptische Vorrichtung 1 in der Ausbildung als passives System zur reinen Orientierungserfassung. Ein Beispiel einer haptischen Vorrichtung 1' mit Kraftrückkopplung als aktives System für Force-Feedback oder andere haptische Zwecke ist in Fig. 2 **bis** 6 in unterschiedlichen Ansichten dargestellt. Vorliegend wird unter dem Begriff "passiv" verstanden, dass von außen, beispielsweise durch einen Benutzer, aufgebrachte Kräfte bzw. Drehmomente eine Bewegung entsprechend dem jeweiligen Freiheitsgrad hervorrufen können. Mit dem Begriff "aktiv" wird ein System mit einem oder mehreren Aktoren bezeichnet, die mittels Energiezufuhr kontrollierte Kräfte bzw. Drehmomente entsprechend einem jeweiligen Freiheitsgrad dem Benutzer anzeigen.

In beiden gezeigten Ausführungen weist die haptische Vorrichtung 1, 1' ein Handstück 10, das entsprechend entweder als Stellteil zur passiven Orientierungserfassung oder aktiven Kraftrückkopplung dient, eine Basisstruktur 5 und eine Handgelenkstruktur auf, die das Handstück 10 mit der Basisstruktur 5 verbindet. Die Basisstruktur 5 könnte im Raum fixiert oder am Ende einer Positionierungsvorrichtung angebracht sein, die beispielsweise translatorische Freiheitsgrade bereitstellt.

Die Handgelenkstruktur der haptische Vorrichtung 1 in Fig. 1 und der haptischen Vorrichtung 1' mit Kraftrückkopplung in Fig. 2 **bis** 6 umfasst eine Kardanikvorrichtung 2, die in dem Handstück 10 zumindest teilweise aufgenommen ist. Das Handstück 10, das hier eine abgeschnittene Kugelform aufweist, hat dazu eine entsprechend zur Aufnahme der Kardanikvorrichtung 2 ausgebildete Aufnahmeöffnung 10.1. Die Kardanikvorrichtung 2 stellt zwei orthogonale, sich schneidende Schwenkachsen A, B für zwei rotatorische Freiheitsgrade des Handstücks 10 bereit, die hier als Rollen r und Gieren g definiert werden. Außerdem ist die Kardanikvorrichtung 2 mit einer Betätigungsstange 6 verbunden, die dazu an einem Ende ein Kopplungsende 6.2 aufweist. Die Betätigungsstange 6 ist um ihre Längsachse C rotierbar, um für den dritten rotatorischen Freiheitsgrad des Handstücks 10 zu sorgen, der hier als Nicken n definiert ist. Über das Kopplungsende 6.2 kann die Kardanikvorrichtung 2 und damit das Handstück 10 zusammen mit der Betätigungsstange 6 um die Längsachse C rotieren.

Damit bietet die Kardanikvorrichtung 2 eine vollständige Orientierung im Raum, wobei der etwas eingeschränkte Bewegungsbereich um die zwei Achsen A, B der Kardanikvorrichtung 2 aufgrund der Anordnung innerhalb des Handstücks 10 durch Rotation der Handgelenkstruktur um eine zusätzliche Rotationsachse X an der Basisstruktur 5 erweitert werden kann. Die Einschränkung der Schwenkbereiche der Kardanikvorrichtung 2 um die Schwenkachsen A, B für die zwei rotatorischen Freiheitsgrade Rollen r und Gieren g hängt auch von der Gestaltung des Kopplungsendes 6.2 ab und liegt im dargestellten Beispiel bei +/- 60° in Bezug auf die neutrale Lage des Handstücks 10, in der die Schwenkachse A, B orthogonal zur Längsachse C liegen. Der Bewegungsbereich der Rotation um die Längsachse C gemäß dem dritten Freiheitsgrad Nicken n ist theoretisch unbegrenzt.

Zur Halterung der Betätigungsstange 6 weist die Handgelenkstruktur der haptischen Vorrichtung 1, 1` einen Grundarm 4 mit einem Halterungsabschnitt 4.1 auf. Der mit der Basisstruktur 5 verbundene Grundarm 4 ist starr ausgeführt, sodass durch Form, Länge und Verlauf des Grundarms 4 die Lage und Ausrichtung des Halterungsabschnitts 4.1 in Bezug auf die Basisstruktur 5 festgelegt sind. Damit sind auch Lage und Ausrichtung der von dem Halterungsabschnitt 4.1 gehaltenen Betätigungsstange 6 in Bezug auf die Basisstruktur 5 festgelegt. Der Halterungsabschnitt 4.1 weist in den dargestellten Beispielen am freien Ende des Haltearms 4 ein Lagerauge zur Halterung der Betätigungsstange 6 auf, die dazu drehbar in einem Stangengehäuse 3 gelagert ist, das an dem Halterungsabschnitt 4.1 befestigt ist. Durch den Abstand des Kopplungsendes 6.2 der Betätigungsstange 6 von dem Halterungsabschnitt 4.1 wird dann die Lage des Handstücks 10 in Bezug zu der Basisstruktur 5 bestimmt.

Der Grundarm 4 kann, wie in Fig. 1 angedeutet, drehbar um die zusätzliche Rotationsachse X an der Basisstruktur 5 angeordnet sein. Es kann eine entsprechende zusätzliche Rotationsachse X auch bei einer haptischen Vorrichtung 1' mit Kraftrückkopplung wie in **Fig. 2 bis** 6 vorgesehen werden. 5, 2. Die bewegliche Anordnung des Grundarms 4 an der Basisstruktur 5 sorgt für einen erweiterten Bewegungsraum der Achsen A und B, wobei das Ausmaß der jeweiligen Erweiterung von der Ausformung des Grundarms 4 abhängig ist. Diese Erweiterung des Bewegungsraums ist allerdings mit einer parasitären Positionsänderung verbunden und weist eine komplexe Dynamik abhängig von Softwareimplementierung und Nutzungsverhalten auf. Daher kann vorgesehen sein, dass dieser zusätzliche Freiheitsgrad der Rotation um die Rotationsachse X nur in der Nähe der Extremlagen aktiviert wird, d. h., dass eine Rotation des Grundarms 4 um die Rotationsachse X an der Basisstruktur 5 innerhalb eines "Normalbereichs" blockiert ist. So ist eine quasi-statische Nutzung durch starke Achsgewichtung möglich.

Alternativ kann der Grundarm 4 unbeweglich an der Basisstruktur 5 befestigt sein, was vorteilhaft in Bezug auf Dynamik, Kosten und Komplexität der haptischen Vorrichtung 1, 1' ist.

Daher stellen der in den Figuren 1 **bis** 6 dargestellten Grundarm 4, bei dem der Halterungsabschnitt 4.1 über einen abgewinkelten Verbindungsabschnitt 4.2 mit einem in einer anderen Ebene abgewinkelten und gebogenen Basisabschnitt 4.3 verbunden ist, und der vorbestimmte Abstand zwischen dem Kopplungsende 6.2 und dem Halterungsabschnitt 4.1 nur eine beispielhafte Ausführung dar, auf die die Erfindung nicht beschränkt sein soll. Denn die Gestaltung des Grundarms 4 einer erfindungsgemäßen haptischen Vorrichtung 1, 1' richtet sich nach der gewünschten Ausrichtung und Lage der Betätigungsstange 6 bzw. der Schwenkachse C in Bezug auf die Basisstruktur 5 und kann daher hinsichtlich der Anzahl, Form, Länge und Abwinkelung der Armabschnitte variieren. Die Lage des Handstücks 10 mit der Kardanikvorrichtung 2 in Bezug auf die Basisstruktur 5 kann dann durch Variation des Abstands zwischen dem Kopplungsende 6.2 und dem Halterungsabschnitt 4.1 festgelegt werden. Durch eine Modifizierung des Grundarms 4 und Variation des vorbestimmten Abstands zwischen dem Kopplungsende 6.2 und dem Halterungsabschnitt 4.1 kann ein großer Bewegungsbereich für das System erreicht werden.

Für die Kardanikvorrichtung 2 der in den Figuren 1 **bis** 6 dargestellten beispielhaften haptischen Vorrichtungen 1, 1' ist das Kopplungsende 6.2 der Betätigungsstange 6 als Kugelabschnitt 6.2 ausgebildet. Dieser ist in einer Kugelpfannenhülse 2.3 gelenkig aufgenommen, die drehfest in dem Handstück 10 angeordnet ist. In der Aufnahmeöffnung 10.1 des Handstücks 10 ist dazu ein Stutzen 10.2 ausgebildet, den die Kugelpfannenhülse 2.3 mit einem Hülsenabschnitt 2.3a umgibt. Zur Aufnahme des Kugelabschnitts 6.2 weist die Kugelpfannenhülse 2.3 einen Kugelpfannenabschnitt 2.3b auf, der mit dem Rand der Aufnahmeöffnung 10.1 abschließt.

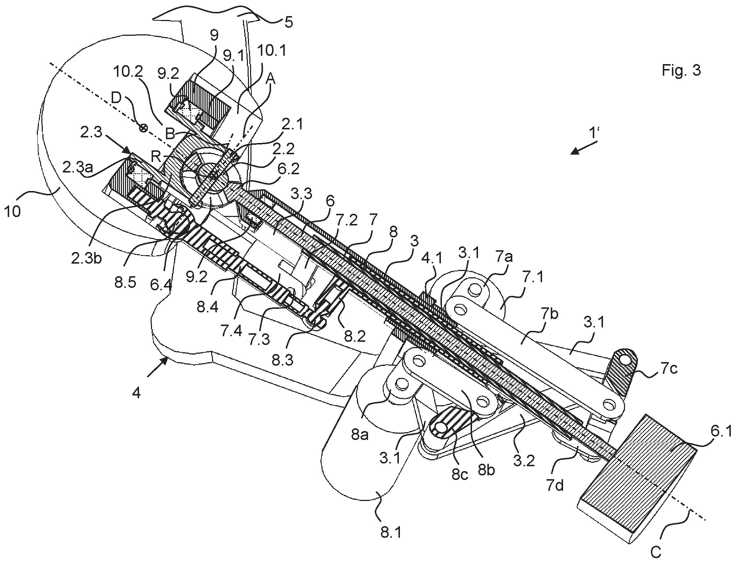

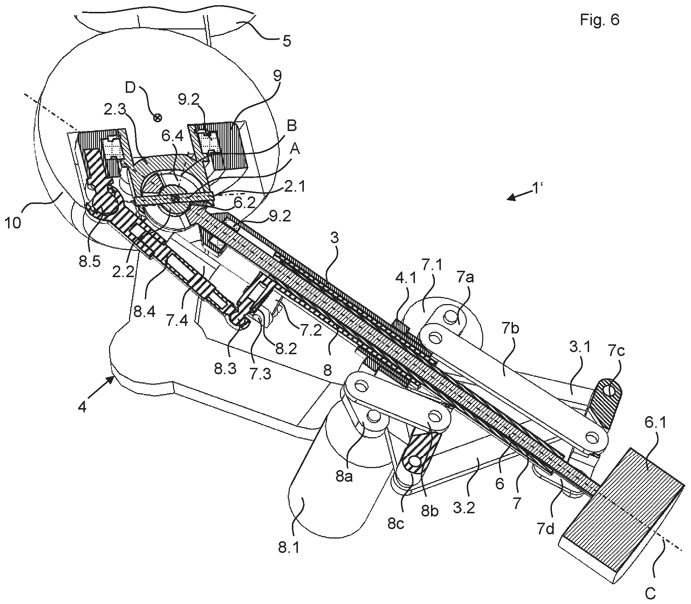

Um die beiden Schwenkachsen A, B für die zwei rotatorischen Freiheitsgrade Rollen r und Gieren g bereitzustellen, weist die dargestellte Kardanikvorrichtung 2 eine Kreuzstrebe 2.1 und ein Zylinderstück 2.2 auf, die mit dem Kugelabschnitt 6.2 gekoppelt sind. Dabei ist das Zylinderstück 2.2 drehbar in einer Zylinderbohrung 6.3 des Kugelabschnitts 6.2 angeordnet, die sich orthogonal zur Längsachse C durch den Kugelabschnitt 6.2 erstreckt. Wie insbesondere in den Schnittdarstellungen von **Fig. 3** **und** **6** gut erkennbar ist, erstreckt sich die Kreuzstrebe 2.1 orthogonal durch das Zylinderstück 2.2, sodass beide Endabschnitte der Kreuzstrebe 2.1 diametral aus dem Zylinderstück 2.2 herausragen. Diese Endabschnitte sind beweglich in zwei diametral im Kugelabschnitt 6.2 ausgebildeten kreisbogenartigen Nutsegmenten 6.4 aufgenommen, die sich entlang einer Ebene erstrecken, die orthogonal zu der Zylinderbohrung 6.3 durch einen Kreismittelpunkt des Kugelabschnitts 6.2 verläuft. Dabei ragen die Endabschnitte der Kreuzstrebe 2.1 auch aus dem Kugelabschnitt 6.2 hinaus und sind drehbar in der Kugelpfannenhülse 2.3 gelagert.

In Fig. 2 **bis** 6 ist ein Beispiel einer haptischen Vorrichtung 1' mit Kraftrückkopplung für Force-Feedback oder andere haptische Zwecke in unterschiedlichen Ansichten und Positionen gezeigt. Die beispielhaft dargestellte haptische Vorrichtung 1' mit Kraftrückkopplung weist Elektromotoren als Aktoren auf, um eine Rückmeldung von Kraft über das Handstück 10 an einen Nutzer auszugeben. Dazu ist die erste Betätigungsstange 6 an einem von dem Kopplungsende 6.2 abgewandten Ende mit einem ersten Motor 6.1 verbunden, der eine Drehbewegung der ersten Betätigungsstange 6 um ihre Längsachse C und damit eine Nick-Drehbewegung des Handstücks 10 entsprechend dem dritten rotatorischen Freiheitsgrad n betätigen kann. Zur Betätigung einer Roll-Drehbewegung des Handstücks 10 um die erste Schwenkachse A der Kardanikvorrichtung 2 entsprechend dem ersten rotatorischen Freiheitsgrad r weist die haptische Vorrichtung 1' einen zweiten Motor 7.1 auf, der über eine erste Kraftübertragungsvorrichtung mit der Kardanikvorrichtung 2 operativ gekoppelt ist. Entsprechend dient ein dritter Motor 8.1, der über eine zweite Kraftübertragungsvorrichtung mit der Kardanikvorrichtung 2 operativ gekoppelt ist, zur Betätigung einer Gier-Drehbewegung des Handstücks 10 um die zweite Schwenkachse B der Kardanikvorrichtung 2 entsprechend dem zweiten rotatorischen Freiheitsgrad g.

Alle drei Motoren 6.1, 7.1, 8.1 sind dabei statisch in Bezug auf den Grundarm 4 angeordnet, wobei auf eine Darstellung etwaiger Halterungen für die Aktoren der Übersichtlichkeit wegen verzichtet wurde. Zur Befestigung der Aktoren an dem Grundarm 4 oder der Basisstruktur 5 oder einer anderen, nicht dargestellten Vorrichtungskomponente kann der Fachmann auf gängige Maßnahmen zurückgreifen. Dass sich die Motoren 6.1, 7.1, 8.1 der haptischen Vorrichtung 1' in Bezug zueinander nicht bewegen, sorgt für eine verbesserte Dynamik des Systems.

Im dargestellten Beispiel handelt es sich bei der ersten und zweiten Kraftübertragungsvorrichtung jeweils um ein Schubstangengestänge; allerdings ist es möglich, dass eine Kraftübertragungsvorrichtung auch auf andere Weise realisiert werden kann, einschließlich, aber nicht beschränkt auf Kabelantriebe, Direktantriebe oder Kombinationen davon. Die Betätigung kann dabei ferner hydraulische, pneumatische, elektromagnetische oder piezoelektrische Aktoren umfassen.

Zur operativen Kopplung der Kardanikvorrichtung 2 mit den Schubstangengestängen (oder anderen Kraftübertragungsvorrichtungen in einer nicht dargestellten Ausführungsform) weist die haptische Vorrichtung 1' einen in dem Handstück 10 angeordneten Befestigungskragen 9 auf, der insbesondere in den Schnittdarstellungen in **Fig. 3** **und** **6** zu sehen ist. Der Befestigungskragen 9 umgibt ringförmig die Kugelpfannenhülse 2.3, bzw. deren Hülsenabschnitt 2.3a und ist mittels eines Lagers 9.2 von einer Drehbewegung der Kardanikvorrichtung 2 und des Handstücks 10 um die Längsachse C entkoppelt. Für diese Entkopplung ist die Aufnahmeöffnung 10.1 in Bezug auf den Befestigungskragen 9 mit Spiel dimensioniert, d. h., dass der Durchmesser der Aufnahmeöffnung 10.1 größer ist als der Außendurchmesser des Befestigungskragens 9, sodass ein Spalt zwischen dem Befestigungskragen 9 und der Innenfläche der Aufnahmeöffnung 10.1 besteht. Die axiale Fixierung des Lagers 9.2 wird außenseitig durch einen Befestigungsring 9.1 und innenseitig durch einen Absatz an dem Hülsenabschnitt 2.3a bereitgestellt.

Außerdem stellt der Befestigungskragen 9 eine gelenkige Verbindung mit den beiden Kraftübertragungsvorrichtungen bzw. Schubstangengestängen zur operativen Kopplung mit der Kardanikvorrichtung 2 bereit. Die beiden Schubstangengestänge sind ähnlich aufgebaut, wobei das erste Schubstangengestänge die Kraft des zweiten Motors 7.1 zur Betätigung einer Roll-Drehbewegung r des Handstücks 10 auf den Befestigungskragen 9 überträgt und das zweite Schubstangengestänge die Kraft des dritten Motors 8.1 zur Betätigung einer Gier-Drehbewegung g des Handstücks 10. Daher werden nachfolgend zur Vereinfachung das erste Schubstangengestänge auch als Roll-Gestänge und das zweite Schubstangengestänge als Gier-Gestänge bezeichnet. Entsprechend werden die jeweiligen Komponenten des Roll- und Gier-Gestänges auch als Roll- bzw. Gier-Komponenten bezeichnet.

Das Roll-Gestänge (erste Schubstangengestänge) umfasst eine zur Längsachse C koaxiale hohlzylindrische Roll-Betätigungsstange 7, die in Längsrichtung linear beweglich auf der bzw. um die erste Betätigungsstange 6 angeordnet ist. Am handstückfernen Ende ist die Roll-Betätigungsstange 7 mit dem zweiten Motor 7.1 über ein Exzentergestänge verbunden, das vier gelenkig verbundene Gestängeabschnitte 7a, 7b, 7c, 7d aufweist, um die Drehbewegung des Motors 7.1 in eine Linearbewegung der Roll-Betätigungsstange 7 umzuwandeln. In bekannter Weise kann dabei eine Unter- oder Übersetzung der Motorexzenterkraft über Variation der Länge bzw. Hebelverhältnisse der Gestängeabschnitte 7a, 7b, 7c, 7d angepasst werden. An ihrem handstücknahen Ende weist die Roll-Betätigungsstange 7 einen Kopplungsabschnitt 7.2 auf, der sich in radialer Richtung bezogen auf die Längsachse C erstreckt. Ein von der Roll-Betätigungsstange 7 beabstandeter Roll-Übertragungsabschnitt 7.4 ist einenends über eine Scharnier-Gelenkverbindung 7.3 mit dem Kopplungsabschnitt 7.2 und anderenends über ein Kugelgelenk 7.5 mit dem Befestigungskragen 9 verbunden.

In ähnlicher Weise umfasst das Gier-Gestänge (zweite Schubstangengestänge) eine zur Längsachse C koaxiale hohlzylindrische Gier-Betätigungsstange 8, die in Längsrichtung linear beweglich die Roll-Betätigungsstange 7 umgibt. Die Gier-Betätigungsstange 8 ist entsprechend kürzer ausgeführt als die Roll-Betätigungsstange 7, sodass beide Enden der Roll-Betätigungsstange 7 aus der Gier-Betätigungsstange 8 herausragen. Die Gier-Betätigungsstange 8 ist an ihrem handstückfernen Ende über ein Exzentergestänge 8a, 8b, 8c, 8d mit dem dritten Motor 8.1 verbunden, um dessen Drehbewegung mittels der vier gelenkig verbundenen Gestängeabschnitte 8a, 8b, 8c, 8d in eine Linearbewegung der Gier-Betätigungsstange 8 mit gewünschter Über- oder Untersetzung zu übertragen.

Selbstverständlich ist es möglich, wenn andere Aktoren eingesetzt werden, die eine lineare Bewegung bereitstellen, wie z. B. ein Linearantrieb oder Hydraulikantrieb, das Exzentergestänge wegfallen und der Aktor direkt oder über eine Unter- oder Übersetzungsstufe mit der jeweiligen Betätigungsstange verbunden werden.

An ihrem handstücknahen Ende weist die Gier-Betätigungsstange 8 ebenfalls einen sich in radialer Richtung erstreckenden Kopplungsabschnitt 8.2 auf, der bezogen auf die Längsachse C um 90° versetzt zu dem Kopplungsabschnitt 7.2 des Roll-Gestänges angeordnet ist. Der Kopplungsabschnitt 8.2 des Gier-Gestänges ist über eine Kugel-Gelenkverbindung 8.3 mit einem Gier-Übertragungsabschnitt 8.4 verbunden, der somit von der Gier-Betätigungsstange 8 beabstandet über ein weiteres Kugelgelenk 8.5 mit dem Befestigungskragen 9 verbunden ist.

Dadurch, dass die Kugel-Gelenkverbindung 8.3 des Gier-Gestänges zwei Freiheitsgrade und die Scharnier-Gelenkverbindung 7.3 des Roll-Gestänges einen Freiheitsgrad aufweist, wird verhindert, dass das System mit den zwei Kraftübertragungsvorrichtungen unbestimmt wird, während gleichzeitig eine vollständige Bewegungsfreiheit des Befestigungskragens 9 gewährleistet ist.

Sowohl die Roll-Betätigungsstange 7 als auch die Gier-Betätigungsstange 8 sind zusammen mit der ersten (Nick-)Betätigungsstange 6 in dem Stangengehäuse 3 angeordnet. Zum Durchtritt der beiden Kopplungsabschnitte 7.2, 8.2 weist das Stangengehäuse 3 an einem handstücknahen Abschnitt in Längsrichtung einen Längsschlitz 3.3 auf, der sich über 90° des Gehäuseumfangs erstreckt. Außerdem hat die Gier-Betätigungsstange 8, die hier die äußerste Betätigungsstange ist, einen Längsschlitz 8.6, der zu dem handstücknahen Ende offen ist, sodass der Roll-Kopplungsabschnitt 7.2 der Gier-Betätigungsstangen 7 bei Annäherung an das handstücknahe Ende der Gier-Betätigungsstange 8 in den Längsschlitz 8.6 eintreten kann. Die Länge des Längsschlitzes 8.6 in der äußersten Betätigungsstange 8 und die Länge des Längsschlitzes 3.3 im Stangengehäuse 3 richten sich nach den Arbeitswegen der Roll- und Gier-Betätigungsstangen 7, 8. Der Längsschlitz 3.3 im Stangengehäuse 3 wird dabei in axialer Richtung beidseitig durch das Stangengehäuse 3 begrenzt, wobei am handgriffnahen Ende in dem Stangengehäuse 3 ein Lager 9.2 angeordnet ist, mit dem die Nick-Betätigungsstange 6 benachbart zu ihrem Kopplungsabschnitt 6.2 drehbar in dem Stangengehäuse 3 gelagert ist.

Bei der in Fig. 3 dargestellten Anordnung des Handstücks 10 in neutraler Lage, in der die Schwenkachsen A, B orthogonal zur Längsachse C liegen, befinden sich die Kopplungsabschnitte 7.2, 8.2 der Roll- und Gier-Betätigungsstangen 7, 8 in ihrer Neutralstellung in der Mitte des jeweiligen Arbeitswegs. Dabei sind die Kopplungsabschnitte 7.2, 8.2 in axialer Richtung entlang der Längsachse C etwas versetzt zueinander angeordnet, wobei der Roll-Kopplungsabschnitt 7.2 näher an dem Handstück 10 steht als der Gier-Kopplungsabschnitt 8.2, weshalb der Gier-Übertragungsabschnitt 8.4 etwas länger ausgebildet ist als der Roll-Übertragungsabschnitt 7.4.

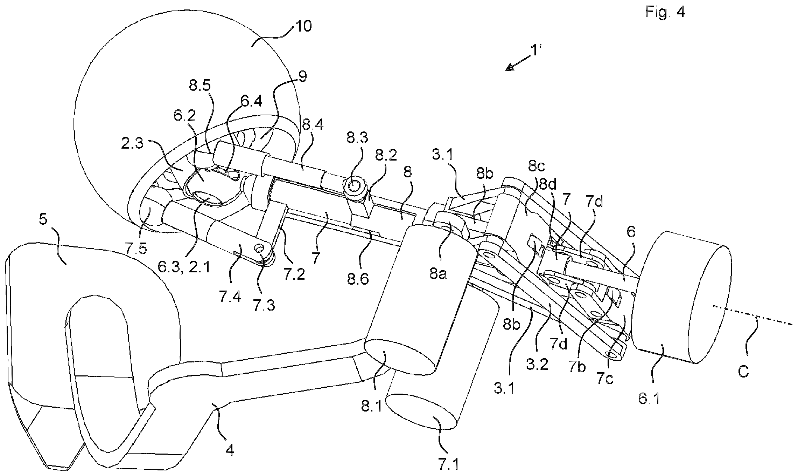

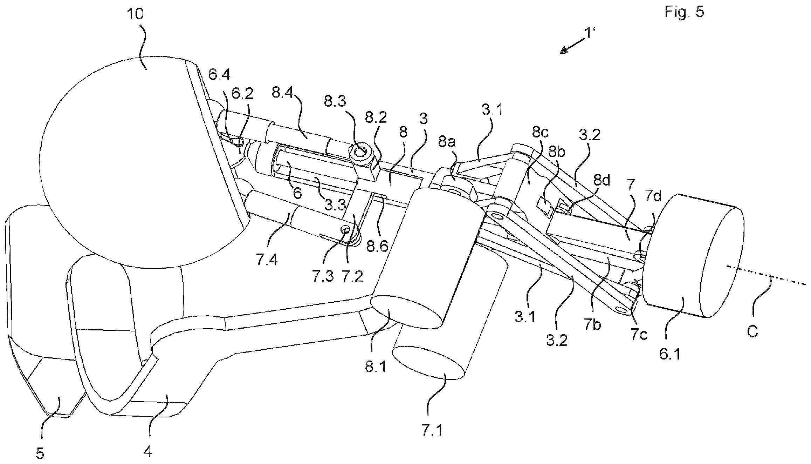

Mit den beiden zueinander orthogonal angeordneten Kopplungsabschnitten 7.2, 8.2 sind die Positionen der Kugelgelenke 7.5, 8.5 des Roll- und Gier-Gestänges an dem Befestigungskragen 9 um 90° in Bezug auf die Längsachse C versetzt. Die Position des Kugelgelenks 7.5 an dem Befestigungskragen 9 wird in Bezug auf die Kardanikvorrichtung 2 so gewählt, dass ein Krafthebel zwischen dem Kugelgelenk 7.5 und dem Rotationspunkt R der Kardanikvorrichtung 2 orthogonal zur Schwenkachse A verläuft, sodass das Kugelgelenk 7.5 hier in der Flucht der Schwenkachse B liegt. Dann führt eine lineare Bewegung der Roll-Betätigungsstange 7 durch Übertragung mit dem Roll-Übertragungsabschnitt 7.4 und dem Kugelgelenk 7.5 zu einer Roll-Drehbewegung r des Befestigungskragens 9 mit dem Handstück 10 um die Schwenkachse A. In **Fig. 4** **und** **5** ist das Handstück 10 in einer um die Schwenkachse A nach innen (von dem Grundarm 4 weg) gerollten Lage und in einer nach außen (zu dem Grundarm 4 hin) gerollten Lage dargestellt, die durch eine lineare Bewegung der Roll-Betätigungsstange 7 entlang der Längsachse C betätigt werden.

Zur Betätigung des Handstücks 10 in die nach innen gerollte Lage (Fig. 4) ist die Roll-Betätigungsstange 7 von dem Motor 7.1 mittels des Exzentergestänges 7a, 7b, 7c, 7d entlang der Längsachse C zum Handstück 10 hin vorgeschoben. Es ist zu sehen, dass sich der Roll-Kopplungsabschnitt 7.2 an dem handstücknahen Axialanschlag des Längsschlitzes 3.3 im Stangengehäuse 3 befindet, während der Gier-Kopplungsabschnitt 8.2 in seiner der neutralen Lage entsprechenden Stellung entlang der Längsachse C verbleibt. Die Vorschubkraft wird von dem Roll-Kopplungsabschnitt 7.2 auf den Roll-Übertragungsabschnitt 7.4 übertragen und wirkt an der Position des Kugelgelenks 7.5 auf den Befestigungskragen 9 ein, sodass sich das Handstück 10 um die Schwenkachse A dreht, die durch die Kreuzstrebe 2.1 der Kardanikvorrichtung 2 bereitgestellt wird.

In Fig. 5 ist die Roll-Betätigungsstange 7 von dem Motor 7.1 mittels des Exzentergestänges 7a, 7b, 7c, 7d entlang der Längsachse C vom Handstück 10 weg zurückgezogen, sodass der Roll-Kopplungsabschnitt 7.2 in den Längsschlitz 8.6 der Gier-Betätigungsstange 8 aufgenommen ist, deren Stellung unverändert ist, wie an der Lage des Gier-Kopplungsabschnitts 8.2 zu erkennen ist. Unter Einwirkung der Rückzugsbewegung der Roll-Betätigungsstange 7, die über den Kopplungs- und Übertragungsabschnitt 7.2, 7.4 auf den Befestigungskragen 9 übertragen wird, wird das Handstück 10 in entgegengesetzter Richtung um die Schwenkachse A bzw. die Kreuzstrebe 2.1 der Kardanikvorrichtung 2 in die nach außen gerollte Lage verschwenkt.

Entsprechend wird die Position des Gier-Kugelgelenks 8.5 an dem Befestigungskragen 9 in Bezug auf die Kardanikvorrichtung 2 so gewählt, dass ein Krafthebel zwischen dem Gier-Kugelgelenk 8.5 und dem Rotationspunkt R der Kardanikvorrichtung 2 orthogonal zur Schwenkachse B verläuft, sodass das Gier-Kugelgelenk 8.5 in der Flucht der Schwenkachse A liegt. Analog zu obiger Beschreibung führt dann eine lineare Bewegung der Gier-Betätigungsstange 8 durch Übertragung mit dem Gier-Kopplungs- und Übertragungsabschnitt 8.2, 8.4 und dem Kugelgelenk 8.5 zu einer Gier-Drehbewegung g des Befestigungskragens 9 mit dem Handstück 10 um die Schwenkachse B.

Aus der in Fig. 3 dargestellten neutralen Lage wird das Handstück 10 in eine nach rechts gegierte Lage überführt, die in Fig. 6 abgebildet ist, indem die Gier-Betätigungsstange 8 von dem Motor 8.1 mittels des Exzentergestänges 8a, 8b, 8c, 8d entlang der Längsachse C zum Handstück 10 hin vorgeschoben wird, bis der Roll-Kopplungsabschnitt 7.2 (der in seiner neutralen Stellung bleibt) im Längsschlitz 8.6 der Gier-Betätigungsstange 8 aufgenommen ist, wie in Fig. 6 durch die relative Stellung der beiden Kopplungsabschnitte 7.2, 8.2 entnehmbar ist. Der Vorschub wird von dem Gier-Kopplungsabschnitt 8.2 auf den Gier-Übertragungsabschnitt 8.4 übertragen und wirkt an der Position des Gier-Kugelgelenks 8.5 auf den Befestigungskragen 9 ein, sodass sich das Handstück 10 mit dem Befestigungskragen 9 um die Schwenkachse B dreht, die durch das Zylinderstück 2.2 der Kardanikvorrichtung 2 bereitgestellt wird.

Der Schnittpunkt der beiden Schwenkachsen A, B als Rotationszentrum R der Kardanikvorrichtung 2 liegt auf der Längsachse C und kann von einem anatomischen Drehpunkt D der Hand eines Benutzers, die das hier kugelförmige Handstücks 10 umgreift, beabstandet sein. Dies gilt sowohl für eine haptische Vorrichtung 1 zur passiven Orientierungserfassung als auch eine haptische Vorrichtung 1', die aktiv für Force-Feedback bzw. Kraftrückkopplung ausgebildet ist. In der Schnittdarstellung von **Fig. 3** **und** **6** wird angenommen, dass der natürliche Drehpunkt D in etwa dem Mittelpunkt des kugelförmigen Handstücks 10 entspricht. Wird das Handstück 10 auf seiner neutralen Lage (Fig. 3), in der der Drehpunkt D auf der Längsachse C liegt, um Schwenkachse A und/oder B verschwenkt, verlässt der Drehpunkt D die Längsachse C, wie in Fig. 6 erkennbar ist, die das Handstück 10 nach einer Gierbewegung nach rechts zeigt. Diese Abweichung wird umso größer, je weiter das Rotationszentrum R vom natürlichen Drehpunkt D der Hand entfernt ist, wenn die Basisstruktur 5 ortsfest im Raum fixiert ist, wobei sich die Lage des Drehpunkts D während der Ausrichtung ändert. Ist die Basisstruktur 5 als Endeffektor an einer Positioniervorrichtung befestigt, so führt ein zunehmender Abstand zwischen dem Rotationszentrum R und dem natürlichen Drehpunkt D der Handbewegung des Benutzers zu einer größer werdenden parasitären Positionsänderung der befestigten Basisstruktur 5. Durch Modifikation des Handstücks 10 hinsichtlich der äußeren Gestaltung für den Umgriff mit der Hand und hinsichtlich der Lage der Kardanikvorrichtung 2 innerhalb des Handstücks 10 kann der Abstand zwischen dem Rotationszentrum R und dem natürlichen Drehpunkt D variiert bzw. minimiert werden, sodass Rotationszentrum R der Drehpunkt D kaum beabstandet voneinander sind und ggf. zusammenfallen.

Es versteht sich, dass die Erfindung nicht auf die im Zusammenhang mit den Figuren beschriebenen beispielhaften Ausführungsformen beschränkt sein soll. Insbesondere sollen nicht nur von den Figuren abweichende Modifikationen in Bezug auf die äußere Gestaltung des Handstücks und der Lage der Kardanikvorrichtung innerhalb des Handstücks, sondern auch Modifikationen des Grundarms hinsichtlich Ausrichtung und Gestaltung sowie der Kraftübertragungsvorrichtungen umfasst sein. Diese Modifikationen betreffen nicht nur die bereits genannten unterschiedlichen Kraftübertragungsvorrichtungen, sondern auch Details der beispielhaft beschriebenen Schubstangengestänge. Der Schutzumfang der Erfindung soll sich auf Modifikationen von einzelnen Komponenten der beschriebenen Schubstangengestänge beziehen, die insbesondere abweichende Anordnungen der Motoren und der Roll- und Gier-Betätigungsstangen jeweils in Bezug zueinander, sowie abweichende Ausführungen der Exzentergestänge und der operativen Kopplung der Roll- und Gier-Betätigungsstangen mit dem Befestigungskragen betreffen.

### BEZUGSZEICHENLISTE

- 1, 1': Haptische Vorrichtung
- 2: Kardanikvorrichtung
- 2.1, 2.2: Kreuzstrebe, Zylinderstück
- 2.3 (a, b): Kugelpfannenhülse (Hülsenabschnitt, Kugelpfannenabschnitt)
- 3: Stangengehäuse
- 3.1, 3.2: Gabelstück, Verbindungsstrebe
- 3.3: Längsschlitz
- 4: Grundarm
- 4.1, 4.2, 4.3: Armabschnitte
- 5: Basisstruktur
- 6: Erste Betätigungsstange (Nicken)
- 6.1: Aktor/Motor
- 6.2, 6.3, 6.4: Kugelabschnitt, Zylinderbohrung, Nutsegment
- 7: Zweite Betätigungsstange (Rollen)
- 7.1: Aktor/Motor
- 7a, b, c, d: Exzentergestängeabschnitte
- 7.2, 7.3: Kopplungsabschnitt, Scharniergelenk
- 7.4, 7.5: Übertragungsabschnitt, Kugelgelenk
- 8: Dritte Betätigungsstange (Gieren)
- 8.1: Aktor/Motor
- 8a, b, c, d: Exzentergestängeabschnitte
- 8.2, 8.3: Kopplungsabschnitt, Kugelgelenk
- 8.4, 8.5: Übertragungsabschnitt, Kugelgelenk
- 8.6: Längsschlitz
- 9: Befestigungskragen
- 9.1: Befestigungsring
- 9.2: Lager
- 10: Handstück
- 10.1, 10.2: Aufnahmeöffnung, Stutzen
- r, g, n: Rotatorische Freiheitsgrade (Rollen, Gieren, Nicken)
- A, B, C: Schwenkachsen (Rollen, Gieren, Nicken)
- X: Zusätzliche Rotationsachse
- R, D: Rotationspunkt Gelenk, Drehpunkt Hand

## Patentansprüche

1. Haptische Vorrichtung (1, 1') mit einem Handstück (10), einer Basisstruktur (5) und einer Handgelenkstruktur, die das Handstück (10) mit der Basisstruktur (5) verbindet und drei rotatorische Freiheitsgrade des Handstücks (10) in Bezug auf die Basisstruktur (5) bereitstellt,
**dadurch gekennzeichnet, dass**
die Handgelenkstruktur aufweist:
- eine Kardanikvorrichtung (2), die in dem Handstück (10) aufgenommen ist und die zwei orthogonale, sich schneidende Schwenkachsen (A, B) aufweist, wobei eine erste Schwenkachse (A) einen ersten rotatorischen Freiheitsgrad (r) des Handstücks (10) bereitstellt, und eine zweite Schwenkachse (B) einen zweiten rotatorischen Freiheitsgrad (g) des Handstücks (10) bereitstellt, und
- eine erste Betätigungsstange (6), die eine Längsachse (C) definiert und an einem Kopplungsende (6.2) mit der Kardanikvorrichtung (2) verbunden ist, wobei die erste Betätigungsstange (6) um ihre Längsachse (C) drehbar ist und einen dritten rotatorischen Freiheitsgrad (n) des Handstücks (10) bereitstellt, und
- einen Grundarm (4), der mit der Basisstruktur (5) verbunden ist und einen Halterungsabschnitt (4.1) aufweist, der die erste Betätigungsstange (6) in einem vorbestimmten Abstand zu dem Kopplungsende (6.2) drehbar gelagert hält.

2. Haptische Vorrichtung (1, 1') nach Anspruch 1,
**dadurch gekennzeichnet, dass**
die erste Schwenkachse (A) der Kardanikvorrichtung (2) durch eine Kreuzstrebe (2.1) bereitgestellt wird, die drehbar in dem Handstück (10) gelagert ist, und
die zweite Schwenkachse (B) durch ein Zylinderstück (2.2) bereitgestellt wird, durch das sich die Kreuzstrebe (2.1) orthogonal erstreckt und mit beiden Enden diametral aus dem Zylinderstück (2.2) hinausragt, wobei
das Kopplungsende (6.2) der ersten Betätigungsstange (6) als Kugelabschnitt (6.2) ausgebildet ist, der aufweist
- eine Zylinderbohrung (6.3), in der das Zylinderstück (2.2) drehbar angeordnet ist, und
- zwei diametrale kreisbogenartige Nutsegmente (6.4), die sich entlang einer zu der Zylinderbohrung (6.3) orthogonalen Ebene erstrecken, die sich durch einen Mittelpunkt des Kugelabschnitts (6.2) erstreckt, wobei die über das Zylinderstück (2.2) hinausragenden Enden der Kreuzstrebe (2.1) in den Nutsegmenten (6.4) aufgenommen sind.

3. Haptische Vorrichtung (1, 1') nach Anspruch 2,
**dadurch gekennzeichnet, dass**
die Kardanikvorrichtung (2) eine Kugelpfannenhülse (2.3) aufweist, die zur drehbaren Lagerung der Kreuzstrebe (2.1) ausgebildet und drehfest in dem Handstück (10) angeordnet ist, wobei der Kugelabschnitt (6.2) der ersten Betätigungsstange (6) in der Kugelpfannenhülse (2.3) gelenkig aufgenommen ist.

4. Haptische Vorrichtung (1, 1') nach zumindest einem der Ansprüche 1 bis 3,
**dadurch gekennzeichnet, dass**
die erste Betätigungsstange (6) drehbar in einem Stangengehäuse (3) gelagert ist, das in dem vorbestimmten Abstand zu dem Kopplungsende (6.2) mit dem Halterungsabschnitt (4.1) des Grundarms (4) verbunden ist,
und/oder
der Grundarm (4) einen gebogenen und/oder gewinkelten Verlauf aufweist, wobei der Halterungsabschnitt (4.1) mit zumindest einem weiteren Armabschnitt (4.2, 4.3) verbunden ist, wobei der Halterungsabschnitt (4.1) und der zumindest eine weitere Armabschnitt (4.2, 4.3) zueinander gewinkelt verlaufen und/oder jeweils eine gerade oder gebogene Form aufweisen.

5. Haptische Vorrichtung (1, 1') nach zumindest einem der Ansprüche 1 bis 4,
**dadurch gekennzeichnet, dass**
die haptische Vorrichtung (1, 1') als eine haptische Vorrichtung (1') mit Kraftrückkopplung ausgebildet ist, wobei die erste Betätigungsstange (6) an einem von dem Kopplungsende (6.2) abgewandten Ende mit einem ersten Aktor (6.1) verbunden ist, um eine Drehbewegung der ersten Betätigungsstange (6) und des Handstücks (10) um die Längsachse (C) entsprechend dem dritten rotatorischen Freiheitsgrad (n) zu betätigen, wobei der erste Aktor (6.1) an dem Grundarm (4) angeordnet ist.

6. Haptische Vorrichtung (1, 1') nach Anspruch 5,
**dadurch gekennzeichnet, dass**
die haptische Vorrichtung (1')
- einen zweiten Aktor (7.1) zur Betätigung einer Drehbewegung der Kardanikvorrichtung (2) und des Handstücks (10) um die erste Schwenkachse (A) entsprechend dem ersten rotatorischen Freiheitsgrad (r) aufweist, wobei der zweite Aktor (7.1) über eine erste Kraftübertragungsvorrichtung mit der Kardanikvorrichtung (2) operativ gekoppelt und an dem Grundarm (4) angeordnet ist, oder
- einen dritten Aktor (8.1) zur Betätigung einer Drehbewegung der Kardanikvorrichtung (2) und des Handstücks (10) um die zweite Schwenkachse (B) entsprechend dem zweiten rotatorischen Freiheitsgrad (g) aufweist, wobei der dritte Aktor (8.1) über eine zweite Kraftübertragungsvorrichtung mit der Kardanikvorrichtung (2) operativ gekoppelt und an dem Grundarm (4) angeordnet ist, oder
- bevorzugt den zweiten Aktor (7.1) und den dritten Aktor (8.1) aufweist, wobei der zweite Aktor (7.1) über die erste Kraftübertragungsvorrichtung und der dritten Aktor (8.1) über die zweite Kraftübertragungsvorrichtung operativ mit der Kardanikvorrichtung (2) zur Betätigung einer Drehbewegung der Kardanikvorrichtung (2) und des Handstücks (10) um die erste Schwenkachse (A) und die zweite Schwenkachse (B) gekoppelt und an dem Grundarm (4) angeordnet sind.

7. Haptische Vorrichtung (1, 1') nach Anspruch 6,
**dadurch gekennzeichnet, dass**
die haptische Vorrichtung (1') einen Befestigungskragen (9) zur operativen Kopplung der ersten Kraftübertragungsvorrichtung und/oder der zweiten Kraftübertragungsvorrichtung mit der Kardanikvorrichtung (2) aufweist, wobei der Befestigungskragen (9) in dem Handstück (10) angeordnet ist und die Kardanikvorrichtung (2) ringförmig umgibt und gelenkig mit der ersten und/oder zweiten Kraftübertragungsvorrichtung verbunden ist, und wobei der Befestigungskragen (9) mittels eines Lagers (9.2) von einer Drehbewegung der Kardanikvorrichtung (2) und des Handstücks (10) um die Längsachse (C) entsprechend dem dritten rotatorischen Freiheitsgrad (n) entkoppelt ist.

8. Haptische Vorrichtung (1, 1') nach Anspruch 7,
**dadurch gekennzeichnet, dass**
die gelenkige Verbindung des Befestigungskragens (9) mit der ersten Kraftübertragungsvorrichtung und mit der zweite Kraftübertragungsvorrichtung eine erste Gelenkverbindung (7.3) der ersten Kraftübertragungsvorrichtung und eine zweite Gelenkverbindung (8.3) der zweiten Kraftübertragungsvorrichtung aufweist, wobei eine der beiden Gelenkverbindungen (7.3; 8.3), die die erste Gelenkverbindung (7.3) und die zweite Gelenkverbindung (8.3) umfassen, einen Freiheitsgrad und die andere der beiden Gelenkverbindungen (7.3; 8.3) zwei Freiheitsgrade bereitstellt.

9. Haptische Vorrichtung (1, 1 ') nach zumindest einem der Ansprüche 6 bis 8,
**dadurch gekennzeichnet, dass**
die erste und/oder die zweite Kraftübertragungsvorrichtung eine mechanische Kraftübertragungsvorrichtung ist, ausgewählt aus einer Gruppe, die zumindest ein Schubstangengestänge und ein Kabelzugsystem umfasst.

10. Haptische Vorrichtung (1, 1') nach Anspruch 9,
**dadurch gekennzeichnet, dass**
die erste und die zweite Kraftübertragungsvorrichtung durch ein erstes und ein zweites Schubstangengestänge bereitgestellt werden,
wobei das erste Schubstangengestänge eine zur Längsachse (C) koaxiale, hohlzylindrische zweite Betätigungsstange (7) aufweist, die in Längsrichtung beweglich um die erste Betätigungsstange (6) angeordnet ist, und das zweite Schubstangengestänge eine zur Längsachse (C) koaxiale, hohlzylindrische dritte Betätigungsstange (8) aufweist, die in Längsrichtung beweglich um die zweite Betätigungsstange (7) angeordnet ist, wobei die zweite Betätigungsstange (7) an beiden Enden aus der dritten Betätigungsstange (8) hinausragt,
wobei die zweite Betätigungsstange (7) an ihrem handstückfernen Ende über ein erstes Exzentergestänge (7a, 7b, 7c, 7d) mit dem zweiten Aktor (7.1) verbunden ist, und die dritte Betätigungsstange (8) an ihrem handstückfernen Ende über ein zweites Exzentergestänge (8a, 8b, 8c, 8d) mit dem dritten Aktor (8.1) verbunden ist,
und wobei
die zweite Betätigungsstange (7) an ihrem handstücknahen Ende einen sich in radialer Richtung erstreckenden ersten Kopplungsabschnitt (7.2) aufweist, der über eine erste Gelenkverbindung (7.3) mit einem ersten Übertragungsabschnitt (7.4) verbunden ist, der beabstandet zu der zweiten Betätigungsstange (7) angeordnet ist und über ein erstes Kugelgelenk (7.5) mit der Kardanikvorrichtung (2) verbunden ist, und die dritte Betätigungsstange (8) an ihrem handstücknahen Ende einen zweiten Kopplungsabschnitt (8.2) aufweist, der sich in radialer Richtung um 90° versetzt zu dem ersten Kopplungsabschnitt (7.2) erstreckt und über eine zweite Gelenkverbindung (8.3) mit einem zweiten Übertragungsabschnitt (8.4) verbunden ist, der beabstandet zu der dritten Betätigungsstange (8) angeordnet ist und über ein zweites Kugelgelenk (8.5) mit der Kardanikvorrichtung (2) verbunden ist.

11. Haptische Vorrichtung (1, 1') nach Anspruch 10,
**dadurch gekennzeichnet, dass**
die zweite und die dritte Betätigungsstange (7; 8) zusammen mit der ersten Betätigungsstange (6) in dem Stangengehäuse (3) angeordnet sind, wobei das Stangengehäuse (3) an einem handstücknahen Abschnitt in Längsrichtung zumindest einen Längsschlitz (3.3) aufweist, durch den sich der erste und der zweite Kopplungsabschnitt (7.2; 8.2) erstrecken.

12. Haptische Vorrichtung (1, 1') nach zumindest einem der Ansprüche 1 bis 11,
**dadurch gekennzeichnet, dass**
der Grundarm (4) unbeweglich oder drehbar um eine zusätzliche Rotationsachse (X) mit der Basisstruktur (5) verbunden ist, wobei
die Basisstruktur (5) aus einer Gruppe ausgewählt ist, die eine Basisstruktur (5) zur ortsfesten Anordnung im Raum und eine als Endeffektor ausgebildete Basisstruktur (5) zur Anordnung an einem Ende einer Positionierungsvorrichtung umfasst.
